# EUROPEAN PATENT APPLICATION

(11) **EP 3 165 256 A1**
(43) Date of publication of application: **10.05.2017**
(21) Application number: 16196889.6
(22) Date of filing: 02.11.2016
(51) Int. Cl.: A61Q 3/02, A61K 8/73

(54) **A DECORATIVE POLISH COMPOSITION FOR NAILS AND A METHOD OF DECORATING NAILS WITH THE POLISH COMPOSITION**

(30) Priority: 05.11.2015 IT UB20155181
(71) Applicant: Chromavis S.p.A., 20122 Milano (IT)
(72) Inventor: Fularska, Anna, 06-300 Przasnysz (PL); Skalkowska, Justyna, 06-400 Ciechanów (PL); Rogalska, Renata, 06-445 Strzegowo (PL); Praczuk, Sandra, 16-001 Ksiezyno (PL)
(74) Representative: Vanosi, Adelio Valeriano

(57) **Abstract**

Decorative polish compositions for nails are disclosed, as well as a kit comprising the same, and a method of decorating nails with said compositions.

## Description

### FIELD OF THE INVENTION

The present invention relates to decorative polish compositions for nails, to a kit comprising the same, and to a method of decorating nails with said compositions.

### STATE OF THE ART

The prior art for decorating fingernails provides for the application of a layer of coloured, transparent, pearly, etc. nail polish on the nails using a suitable brush.

In order to obtain special effects, after applying the coloured base polish, artistic, geometric or pattern designs are often drawn on the polish using a thin brush to revive the polish with pearlescent, iridescent or glittered visual effects by applying a further polish layer which generates such effects.

Nail polishes are also known which provide for the application of a transparent base polish layer which reinforces and regenerates the nail, and then a coloured layer which gives the desired colour to the nail, on top of which a top coat may be added, which acts as a fixer, shining aid and staying power enhancer.

With changing fashion, consumers, who are increasingly demanding, are looking for novel make-up products which confer original or specific make-up effects. A need therefore remains to have available a polish which, when applied to nails, results in a make-up effect different from those of the smooth, continuous and homogeneous films typically obtained with the commercially available products.

It is the object of the present invention to provide a decorative polish for nails and a method of decorating them by means of said polish which allows to achieve original and specific make-up effects.

### SUMMARY OF THE INVENTION

This and other objects are achieved by a decorative polish composition for nails and by a method of decorating them by means of said composition, implemented according to the technical teachings of the appended claims.

In a further aspect, the present invention relates to a kit comprising at least one decorative polish composition for nails.

In another aspect, the present invention relates to the use of the decorative polish composition as a nail base coat polish and as a nail top coat polish.

The characteristics and the advantages of the present invention will become apparent from the following detailed description, from the working examples provided for illustrative purposes, and from the accompanying Figures, wherein:
Figure 1 shows the final result obtained by means of the method of Example 1;
Figure 2 shows the final result obtained by means of the method of Example 2;
Figure 3 shows the final result obtained by means of the method of Example 3; and
Figure 4 shows the final result obtained by means of the method of Example 4.

### DETAILED DESCRIPTION OF THE INVENTION

The decorative polish composition of the present invention comprises at least a film forming agent, at least a plasticizer, at least a solvent, at least a rheological agent, and at least a colouring agent, further comprising a silicone-modified polysaccharide, as an active agent for obtaining original and specific make-up effects on nails.

Suitable silicone-modified polysaccharides are those described in Japanese Unexamined Patent Publication (Kokai) No. 10-29910, wherein said silicone-modified polysaccharides have the following formula (I): 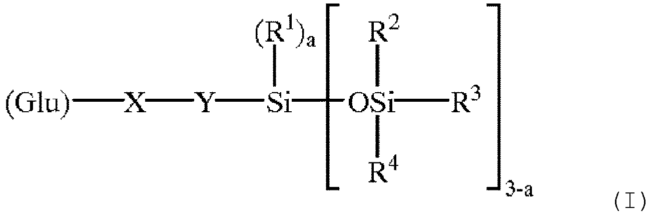 wherein Glu is a polysaccharide moiety, X is a bivalent bond group, Y is a bivalent aliphatic group, R1 is a C1 to C8 monovalent organic group, R2, R3, and R4 independently of one another denote a C1 to C8 monovalent organic group or siloxy group of -OSiR5R6R7 where R5, R6, and R7 independently of one another are C1 to C8 monovalent organic groups. Finally, 'a' denotes 0, 1, or 2.

In formula (I), Glu denotes a polysaccharide moiety, which is preferably a moiety of cellulose, hemicellulose, gum arabic, tragacanth gum, tamarind gum, pectin, starch, mannan, guar gum, locust bean gum, quince seed gum, alginic acid, carrageenan, agar, xanthane gum, dextran, pullulan, chitin, chitosan, hyaluronic acid, chondroitin sulfuric acid, or a derivative thereof, such as carboxymethylated derivatives, sulfate derivatives, phosphated derivatives, methylated derivatives, ethylated derivatives, addition derivatives of alkylene oxide such as ethylene oxide or propylene oxide, acylated derivatives, and cationated derivatives. In preferred embodiments, said polysaccharide compound is pullulan.

In particularly preferred embodiments, said silicone-modified polysaccharide is silicone-modified pullulan of the following formula (II): 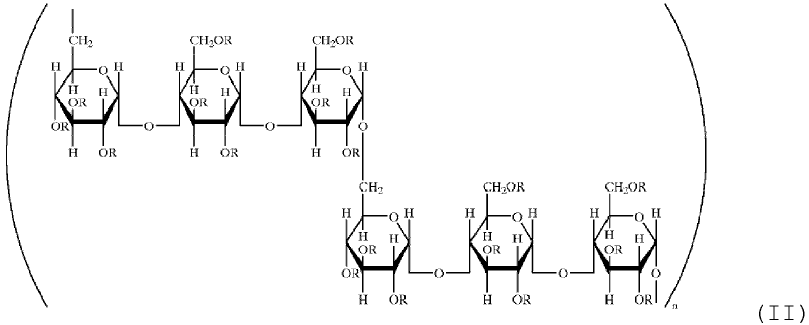 wherein R denotes a hydrogen atom or [ (CH₃) ₃SiO] ₃Si (CH₂) ₃NHCO group, as described in Japanese Unexamined Patent Publication (Kokai) No. 8-134103.

The ratio of bonds differs depending on the type of the silicone compound and polysaccharide compound, but the average number of bonds (i.e., substitution degree) of the silicone compound per unit component sugar of the polysaccharide compound is preferably 0.5 to 2.5. Note that the above substitution degree is obtained by conversion from the Si content (% by weight) in the silicone-modified polysaccharide compound.

In the decorative polish composition of the present invention, the above silicone-modified polysaccharide compound may be one produced by a usual known method of production. Further, a commercially available one may be included.

Preferably, in the decorative nail polish composition of the invention, said silicone-modified polysaccharide is in an amount up to 10wt% on the composition weight.

More preferably, in the decorative nail polish composition of the invention, said silicone-modified polysaccharide is in an amount up to 7wt% on the composition weight.

In particularly preferred embodiments, in the decorative nail polish composition of the invention, said silicone-modified polysaccharide is in an amount of 0.5-6wt% on the composition weight.

In preferred embodiments, the decorative nail polish composition of the invention further comprises an alkane selected from the group consisting of linear or branched C7-C16 alkanes and mixtures thereof.

The more preferred alkane is isododecane.

In other preferred embodiments, the decorative nail polish composition of the invention further comprises a cyclic siloxane selected from the group consisting of hexamethylcyclotrisiloxane (D3), octamethylcyclotetrasiloxane (D4), decamethylcyclopenta-siloxane (D5), dodecamethylcyclohexasiloxane (D6), and mixtures thereof.

The more preferred cyclic siloxane is decamethylcyclopentasiloxane (D5), shortly referred to as 'cyclopentasiloxane'.

It has been found that said silicone-modified polysaccharide works as an active agent for obtaining original and specific make-up effects on nails in a wide range of viscosity, so that the decorative nail polish composition of the invention can have a viscosity of 0.1 Pa*s to 5.0 Pa*s. The viscosity of nail polish can be measured by using a viscometer. The preferred viscometer is Brookfield and rotating viscometer. The resulting measurement is taken at 60 rpm and at 25°C. Therefore, preferably, the decorative nail polish composition of the invention has a Brookfield viscosity of 0.1 Pa*s to 5.0 Pa*s at 60 rpm and 25°C.

The composition of the invention can be a nitrocellulose-based nail polish, a water-based nail polish or a sprayable nail polish.

Nail polishes also contain at least a film forming agent, at least a plasticizer, at least a solvent, at least a rheological agent, and at least a colouring agent.

For the purposes of the present invention, preferably, a nail polish comprises:
7-28wt% of at least a film forming agent,
3-20wt% of at least a plasticizer,
45-90wt% of at least a solvent,
0.5-6wt% of at least a rheological agent, and
up to 20wt% of at least a colouring agent,
on the weight of the nail polish.

More preferably, a nail polish comprises:
11-26wt% of at least a film forming agent,
5-10wt% of at least a plasticizer,
60-75wt% of at least a solvent,
1-3wt% of at least a rheological agent, and
1-10wt% of at least a colouring agent,
on the weight of the nail polish.

A primary ingredient in nitrocellulose-based nail polish is nitrocellulose (cellulose nitrate - cellulose origin can be cotton linters and/or wood pulp). Nitrocellulose can be purchased in various viscosities to match the desired viscosity of the final product.

Nitrocellulose acts as a film forming agent. For nail polish to work properly, a hard film must form on the exposed surface of the nail, but it cannot form so quickly that it prevents the material underneath from drying. By itself or used with other functional ingredients, the nitrocellulose film is brittle and adheres poorly to nails.

Preferably, nitrocellulose is in an amount of 5 to 20wt% on the composition weight, more preferably about 6% to about 20%, most preferably about 13% to about 16%.

Preferably, nitrocellulose is a nitrocellulose of RS types with 11,5-12,4% nitrogen content (other types can be used for example -RS ½ second, -RS ¼ second,-RS 5/6 second), or a mixture thereof.

Other suitable film-forming agents are:
- cellulose film-forming derivatives (cellulosic esters, such as cellulose acetate, cellulose acetate butyrate, cellulose acetate propionate, cellulose ethers and mixtures thereof)
- Synthetic polymers (polyesters, epoxy resins, acrylates copolymer, (meth)acrylic resins, vinyl resins, poly(vinyl alcohol), crosslinked poly(vinyl alcohol), styrene acrylates, polybutene, VP/VA copolymer, polyurethane, hydrogenated polycyclopentadiene, etc.)
- Silicone resins (trimethylsiloxysilicate, polymethylsilsesquioxane, C26-28 Alkyl Dimethicone)
- Ether-based resins (polyvinyl stearyl ether, etc.),
but preferably those indicated above in parentheses.

Any water-dispersible variants of each substance are preferably selected. However, water-dispersible acrylates copolymers are highly preferred.

Plasticizers are added to improve flexibility and mechanical resistance of the film. Suitable plasticizers are castor oil, amyl and butyl stearate, citrates (such as acetyl triethyl citrate, acetyl tributyl citrate, acetyl trihexyl citrate, triethyl citrate), adipates, sebacates, sucrose acetate isobutyrate, sucrose benzoate, glycol esters, fatty acids, camphor, 2,2,4-trimethyl-1,3-pentanediol diisobutyrate, and trimethylpentanediyl dibenzoate.

The colouring agents and other components of nail polish are typically contained within one or more solvents, until the polish is applied. After application, the solvent must be able to evaporate. Butyl acetate, isobutyl acetate, ethyl acetate, propyl acetate, isopropyl acetate, acetone, and isopropyl alcohol are the most commonly used solvents, but silicone-based solvents such as silanes and siloxanes are also suitable. Other suitable solvents are amyl acetate, heptane, 1-butanol, 2-butyl alcohol, methyl ethyl ketone, and water.

Finally, the nail polish can contain also a colouring agent. Choice of pigment and its ability to be well dispersed and properly suspended is essential for producing a good quality product.

In addition to usual colouring pigments, other colour tones can be added depending upon the colour, tone, and hue of the desired product. Natural and synthetic Mica-based products, borosilicates, bismuth oxychloride called 'pearl essence' are white, iridescent or coloured common additives, that can be mixed also with gold, silver, and bronze tones.

The nail polish can also contain at least a rheological agent, which may act to thicken the composition in order to suspend the colouring agents (such as mainly pigments and pearls), while saving a better spreading on the nail. The classic rheological agents used in nail polish are organically treated bentonite and hectorite. Other suitable rheological agents are silica, and thixotropic alkyds.

The decorative polish as above described is in a liquid form at room temperature and can be directly applicable on nails, for example by using a brush or by using a spray applicator.

The spray applicator may be a traditional aerosol. In this case, the decorative polish composition further comprises a propellant. The propellant, preferably liquefied, and the decorative liquid polish are mixed in the following percentages:
- Propellant from 60 to 80%
- Decorative liquid polish from 20 to
40%, thus obtaining a decorative spray polish.

In this case, the above-described product is contained in a chamber of a pressurized spray cylinder of conventional type, preferably made of aluminum, provided with a pressure-opening valve, directly in communication with the chamber. On a hollow stem of the valve there is fixed an aerosol vaporizer cap which atomizes and distributes the product when the valve is pressed.

The propellant used in the example described above belongs to one of the following chemical classes:
- hydrocarbons (butane, propane, etc.)
- ethers (dimethyl ether)
- halogen compounds (difluoroethane)

The preferred substances are indicated in parentheses.

It should be noted that all the percentages are expressed by weight on the weight of the decorative nail polish composition.

In some embodiments, the decorative polish composition of the present invention consists essentially of at least a film forming agent, at least a plasticizer, at least a solvent, at least a rheological agent, at least a colouring agent, an alkane or a cyclic siloxane, and a silicone-modified polysaccharide, as above described. The expression "consists essentially of" means that said silicone-modified polysaccharide is the only active agent in the composition for obtaining original and specific make-up effects on nails, such as a "stone marble" pattern on nails or an "orange peel" effect.

In other embodiments, the decorative polish composition of the present invention consists of at least a film forming agent, at least a plasticizer, at least a solvent, at least a rheological agent, at least a colouring agent, an alkane or a cyclic siloxane, and a silicone-modified polysaccharide, as above described.

In a further aspect, the present invention relates to the use of the decorative nail polish composition for decorating nails.

As it will be clear from the following working Examples, the composition of the invention can be used directly on nails or can be used together with other nails polishes.

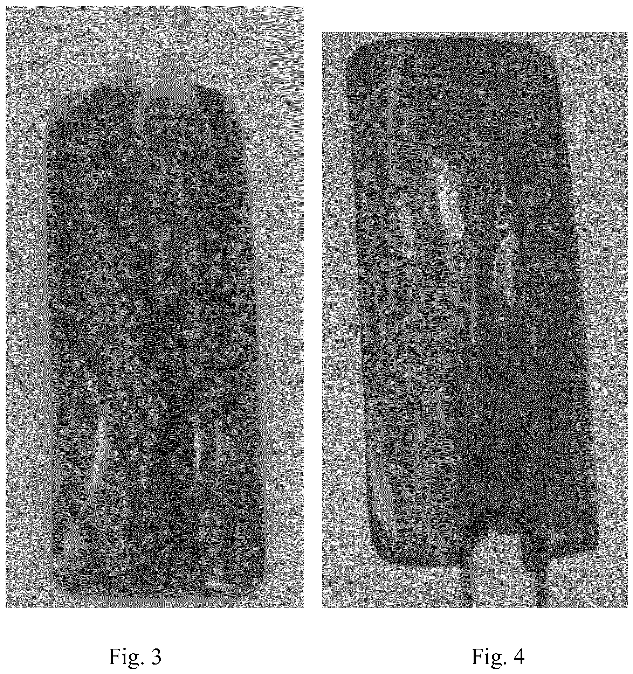

When used directly on nails, the decorative nail polish composition of the invention allows to obtain original and specific make-up effects on nails, especially an "orange peel" pattern on nails (see Figure 4).

In a further aspect, the present invention relates to the use of the decorative nail polish composition as a nail base coat polish.

In another aspect, the present invention relates to the use of the decorative nail polish composition as a nail top coat polish.

In an additional aspect, the present invention relates to a kit of nail polishes, the kit comprising a base coat polish and a top coat polish, wherein:
i) said base coat polish is the decorative nail polish composition of the invention, or
ii) said top coat polish is the decorative nail polish composition of the invention, or
iii) both base coat polish and top coat polish are the decorative nail polish composition of the invention,
and wherein the base coat polish and the top coat polish comprise different colouring agents.

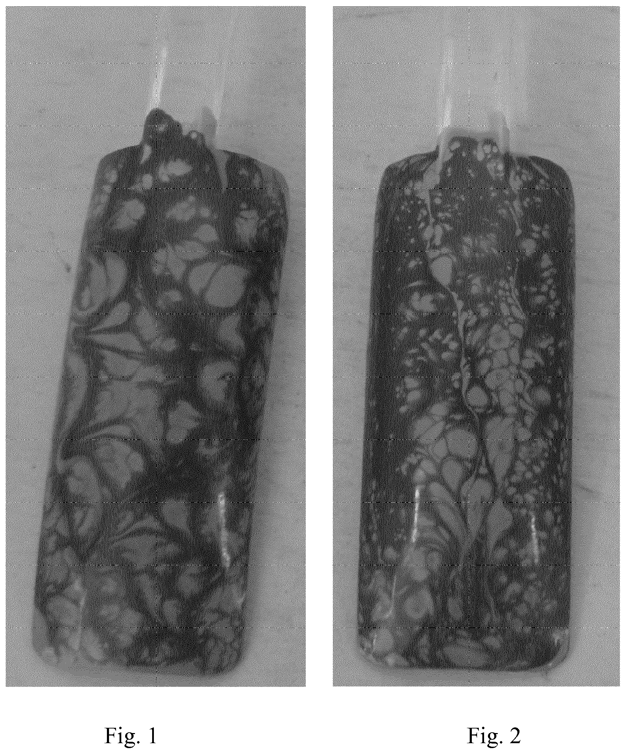

In fact, as widely shown in the following working Examples, it should be appreciated that the decorative nail polish composition of the invention allows to obtain original and specific make-up effects on nails, especially a "stone marble" pattern on nails, either when used as a base coat, or as a top coat, or as both (see Figures 1-3).

The base coat polish and/or the top coat polish of the kit which are not according to the present invention, can be commercially available nail polishes.

In this regard, the present invention also concerns a method for obtaining said original and specific make-up effects on nails, the method comprising the steps of:
a) applying a base coat polish on nails, and
b) applying a top coat polish before the base coat polish dries,
   wherein:
   i) said base coat polish is the decorative nail polish composition of the invention, or
   ii) said top coat polish is the decorative nail polish composition of the invention, or
   iii) both base coat polish and top coat polish are the decorative nail polish composition of the invention,
and wherein the base coat polish and the top coat polish comprise different colouring agents.

As said above, the base coat polish and/or the top coat polish of the kit which are not according to the present invention, can be commercially available nail polishes.

In fact, it has been surprisingly found that the application of the top coat on the base coat when the latter is still wet, i.e. not yet dried, allows to obtain the "stone marble" pattern on nails, either when the decorative nail polish composition of the invention used as a base coat, or as a top coat, or as both.

Without wishing to be bound by any theory, it is believed that the hydrophilic-hydrophobic nature of the silicone-modified polysaccharide changes the surface tension between the two wet polish layers, i.e. base coat and top coat layers, thus resulting in the "stone marble" pattern on nails.

In preferred embodiments, the step b) of the application of the top coat polish is performed less than 30 seconds from the step a) of the application of the base coat polish.

In other preferred embodiments of the kit and of the method, the option ii) is selected, wherein said top coat polish is the decorative nail polish composition of the invention. In fact, it has been found that the final "stone marble" is easier to achieve as the top coat made of the decorative polish composition of the invention allows better spreading on the nail and needs no additional layers or corrections.

The final effect of the "stone marble" pattern on nails can be varied by varying different colouring agents for the base coat polish and the top coat polish.

It should be understood that all the aspects identified as preferred and advantageous for the decorative nail polish composition are to be deemed as similarly preferred and advantageous also for the uses, the kit and method of application of the same.

It should be also understood that all the combinations of preferred aspects of the decorative nail polish composition of the invention, as well as of uses, the kit and method of application, as above reported, are to be deemed as hereby disclosed.

Below are working examples of the present invention provided for illustrative purposes.

### EXAMPLES

### Example 1.

The following compositions were prepared:

| **Base Coat Nail Polish** | **[wt%]** |
|---|---|
| Bentonite | 1.5 |
| ½ sec Nitrocellulose | 11 |
| Acetyl tributyl citrate | 6 |
| Polyester resin | 12 |
| Ethyl acetate | 31 |
| Butyl acetate | 25 |
| Isopropyl alcohol | 5 |
| Titanium dioxide CI 77891 | 3.5 |
| Isododecane (and) Trimethylsiloxysilylcarbamoyl pullulan (Trade name: TSPL-30-ID-F, commercially available from Shin-Etsu) | 5 |
| Total | 100 |

| **Top Coat Nail Polish** | **[wt%]** |
|---|---|
| Bentonite | 2 |
| ½ sec Nitrocellulose | 11 |
| Acetyl tributyl citrate | 6 |
| Sucrose Acetate Isobutyrate | 1 |
| Polyester resin | 9 |
| Acrylic resin | 2 |
| Ethyl acetate | 29 |
| Butyl acetate | 31 |
| Isopropyl alcohol | 7.5 |
| Red 6 lake CI 15850 | 1.5 |
| Total | 100 |

The top coat polish was applied with a brush before the base coat polish was dried, thus obtaining the "stone marble" effect shown in Figure 1.

### Example 2.

The following compositions were prepared:

| **Base Coat Nail Polish** | **[wt%]** |
|---|---|
| Silica | 2 |
| ½ sec Nitrocellulose | 14 |
| Acetyl tributyl citrate | 6 |
| Trimethyl pentanyl diisobutyrate | 2 |
| Polyester resin | 9 |
| Ethyl acetate | 40 |
| Butyl acetate | 14 |
| Isopropyl alcohol | 9 |
| Mica & Iron Oxides CI 77491 | 4 |
| Total | 100 |

| **Top Coat Nail Polish** | **[wt%]** |
|---|---|
| Bentonite | 2 |
| ½ sec Nitrocellulose | 12 |
| Acetyl tributyl citrate | 5 |
| Sucrose Acetate Isobutyrate | 1 |
| Polyester resin | 9 |
| Acrylic resin | 1 |
| Ethyl acetate | 29 |
| Butyl acetate | 31 |
| Isopropyl alcohol | 7.5 |
| Red 6 lake CI 15850 | 1.5 |
| Isododecane (and) Trimethylsiloxysilylcarbamoyl pullulan (Trade name: TSPL-30-ID-F, commercially available from Shin-Etsu) | 1 |
| Total | 100 |

The top coat polish was applied with a brush before the base coat polish was dried, thus obtaining the "stone marble" effect shown in Figure 2.

### Example 3.

The following compositions were prepared:

| **Base Coat Nail Polish** | **[wt%]** |
|---|---|
| Hectorite | 1.5 |
| ½ sec Nitrocellulose | 11 |
| Acetyl tributyl citrate | 7 |
| Polyester resin | 12 |
| Ethyl acetate | 34 |
| Butyl acetate | 22 |
| Isopropyl alcohol | 5 |
| Titanium dioxide CI 77891 | 3.5 |
| Isododecane (and) Trimethylsiloxysilylcarbamoyl pullulan (Trade name: TSPL-30-ID-F, commercially available from Shin-Etsu) | 4 |
| Total | 100 |

| **Top Coat Nail Polish** | **[wt%]** |
|---|---|
| Hectorite | 1.4 |
| ½ sec Nitrocellulose | 12 |
| Acetyl tributyl citrate | 6 |
| Polyester resin | 9 |
| Acrylic resin | 1 |
| Ethyl acetate | 28 |
| Butyl acetate | 31.6 |
| Isopropyl alcohol | 7.5 |
| Red 6 lake CI 15850 | 1.5 |
| Isododecane (and) Trimethylsiloxysilylcarbamoyl pullulan (Trade name: TSPL-30-ID-F, commercially available from Shin-Etsu) | 2 |
| Total | 100 |

The top coat polish was applied with a brush before the base coat polish was dried, thus obtaining the "stone marble" effect shown in Figure 3.

### Example 4.

The following composition was prepared:

| **Nail Polish** | **[wt%]** |
|---|---|
| Bentonite | 1.5 |
| ½ sec Nitrocellulose | 14 |
| Acetyl tributyl citrate | 6 |
| Polyester resin | 12 |
| Ethyl acetate | 31 |
| Butyl acetate | 25 |
| Isopropyl alcohol | 5 |
| Titanium dioxide CI 77891 | 3.5 |
| Isododecane (and) Trimethylsiloxysilylcarbamoyl pullulan (Trade name: TSPL-30-ID-F, commercially available from Shin-Etsu) | 2 |
| Total | 100 |

When used directly on nails, the decorative nail polish composition was applied with a brush directly on nails thus obtaining the "orange peel" effect shown in Figure 4.

### Example 5.

The following compositions were prepared:

| **Base Coat Nail Polish** | **[wt%]** |
|---|---|
| Bentonite | 1.5 |
| ½ sec Nitrocellulose | 11 |
| Acetyl tributyl citrate | 6 |
| Polyester resin | 12 |
| Ethyl acetate | 31 |
| Butyl acetate | 25 |
| Isopropyl alcohol | 5 |
| Titanium dioxide CI 77891 | 3.5 |
| Cyclopentasiloxane (and) Trimethylsiloxysilylcarbamoyl pullulan (Trade name: TSPL-30-D5, commercially available from Shin-Etsu) | 5 |
| Total | 100 |

| **Top Coat Nail Polish** | **[wt%]** |
|---|---|
| Bentonite | 2 |
| ½ sec Nitrocellulose | 11 |
| Acetyl tributyl citrate | 6 |
| Sucrose Acetate Isobutyrate | 1 |
| Polyester resin | 9 |
| Acrylic resin | 2 |
| Ethyl acetate | 29 |
| Butyl acetate | 31 |
| Isopropyl alcohol | 7.5 |
| Red 6 lake CI 15850 | 1.5 |
| Total | 100 |

The top coat polish was applied with a brush before the base coat polish was dried, thus obtaining a "stone marble" effect.

### Example 6.

The following compositions were prepared:

| **Base Coat Nail Polish** | **[wt%]** |
|---|---|
| Silica | 2 |
| ½ sec Nitrocellulose | 14 |
| Acetyl tributyl citrate | 6 |
| Trimethyl pentanyl diisobutyrate | 2 |
| Polyester resin | 9 |
| Ethyl acetate | 40 |
| Butyl acetate | 14 |
| Isopropyl alcohol | 9 |
| Mica & Iron Oxides CI 77491 | 4 |
| Total | 100 |

| **Top Coat Nail Polish** | **[wt%]** |
|---|---|
| Bentonite | 2 |
| ½ sec Nitrocellulose | 12 |
| Acetyl tributyl citrate | 5 |
| Sucrose Acetate Isobutyrate | 1 |
| Polyester resin | 9 |
| Acrylic resin | 1 |
| Ethyl acetate | 29 |
| Butyl acetate | 31 |
| Isopropyl alcohol | 7.5 |
| Red 6 lake CI 15850 | 1.5 |
| Cyclopentasiloxane (and) Trimethylsiloxysilylcarbamoyl pullulan (Trade name: TSPL-30-D5, commercially available from Shin-Etsu) | 1 |
| Total | 100 |

The top coat polish was applied with a brush before the base coat polish was dried, thus obtaining a "stone marble" effect.

### Example 7.

The following compositions were prepared:

| **Base Coat Nail Polish** | **[wt%]** |
|---|---|
| Hectorite | 1.5 |
| ½ sec Nitrocellulose | 11 |
| Acetyl tributyl citrate | 7 |
| Polyester resin | 12 |
| Ethyl acetate | 34 |
| Butyl acetate | 22 |
| Isopropyl alcohol | 5 |
| Titanium dioxide CI 77891 | 3.5 |
| Cyclopentasiloxane (and) Trimethylsiloxysilylcarbamoyl pullulan (Trade name: TSPL-30-D5, commercially available from Shin-Etsu) | 4 |
| Total | 100 |

| **Top Coat Nail Polish** | **[wt%]** |
|---|---|
| Hectorite | 1.4 |
| ½ sec Nitrocellulose | 12 |
| Acetyl tributyl citrate | 6 |
| Polyester resin | 9 |
| Acrylic resin | 1 |
| Ethyl acetate | 28 |
| Butyl acetate | 31.6 |
| Isopropyl alcohol | 7.5 |
| Red 6 lake CI 15850 | 1.5 |
| Cyclopentasiloxane (and) Trimethylsiloxysilylcarbamoyl pullulan (Trade name: TSPL-30-D5, commercially available from Shin-Etsu) | 2 |
| Total | 100 |

The top coat polish was applied with a brush before the base coat polish was dried, thus obtaining a "stone marble" effect.

### Example 8.

The following composition was prepared:

| **Nail Polish** | **[wt%]** |
|---|---|
| Bentonite | 1.5 |
| ½ sec Nitrocellulose | 14 |
| Acetyl tributyl citrate | 6 |
| Polyester resin | 12 |
| Ethyl acetate | 31 |
| Butyl acetate | 25 |
| Isopropyl alcohol | 5 |
| Titanium dioxide CI 77891 | 3.5 |
| Cyclopentasiloxane (and) Trimethylsiloxysilylcarbamoyl pullulan (Trade name: TSPL-30-D5, commercially available from Shin-Etsu) | 2 |
| Total | 100 |

When used directly on nails, the decorative nail polish composition was applied with a brush directly on nails thus obtaining an "orange peel" effect.

## Claims

1. A decorative nail polish composition comprising at least a film forming agent, at least a plasticizer, at least a solvent, at least a rheological agent, and at least a colouring agent, further comprising a silicone-modified polysaccharide.

2. The decorative nail polish composition of claim 1, wherein said silicone-modified polysaccharide is in an amount up to 10wt% on the composition weight.

3. The decorative nail polish composition of claim 2, wherein said silicone-modified polysaccharide is in an amount up to 7wt% on the composition weight.

4. The decorative nail polish composition of any one of claims 1-3, wherein said silicone-modified polysaccharide is silicone-modified pullulan of formula (II): 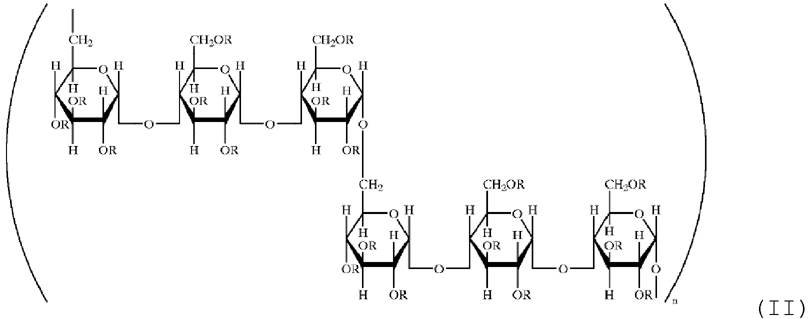 wherein R denotes a hydrogen atom or [(CH₃)₃SiO]₃Si(CH₂)₃NHCO group.

5. The decorative nail polish composition of any one of claims 1-4, further comprising an alkane selected from the group consisting of linear or branched C7-C16 alkanes and mixtures thereof, or a cyclic siloxane selected from the group consisting of hexamethylcyclotrisiloxane, octamethylcyclotetrasiloxane, decamethylcyclopenta-siloxane, dodecamethylcyclohexasiloxane, and mixtures thereof.

6. The decorative nail polish composition of any one of claims 1-5, the composition having a viscosity of 0.1 Pa*s to 5.0 Pa*s.

7. Use of the decorative nail polish composition of any one of claims 1-6, as a nail base coat polish.

8. Use of the decorative nail polish composition of any one of claims 1-6, as a nail top coat polish.

9. Kit of nail polishes, the kit comprising a base coat polish and a top coat polish, wherein:
i) said base coat polish is the decorative nail polish composition of any one of claims 1-6, or
ii) said top coat polish is the decorative nail polish composition of any one of claims 1-6, or
iii) both base coat polish and top coat polish are the decorative nail polish composition of any one of claims 1-6,
and wherein the base coat polish and the top coat polish comprise different colouring agents.

10. Method for obtaining original and specific make-up effects on nails, the method comprising the steps of:
a) applying a base coat polish on nails, and
b) applying a top coat polish before the base coat polish dries,
wherein:
i) said base coat polish is the decorative nail polish composition of any one of claims 1-6, or
ii) said top coat polish is the decorative nail polish composition of any one of claims 1-6, or
iii) both base coat polish and top coat polish are the decorative nail polish composition of any one of claims 1-6,
and wherein the base coat polish and the top coat polish comprise different colouring agents.
